# FASCICULE DE BREVET EUROPEEN

(11) **EP 1 779 841 B1**
(45) Date de publication et mention de la délivrance du brevet: **27.01.2010**
(21) Numéro de dépôt: 06121555.4
(22) Date de dépôt: 29.09.2006
(51) Int. Cl.: A61K 8/49, A61K 8/41, A61K 8/34, A61Q 5/12

(54) **Composition cosmétique comprenant un ester de sorbitan oxyéthyléné, un polymère cationique, un corps gras solide et procédé de traitement cosmétique**
Kosmetische Zusammensetzung enthaltend einen oxyethylierten Sorbitanester, ein kationisches Polymer, einen festen Fettkörper
Cosmetic composition comprising an oxyethylenated sorbitan ester, a cationic polymer and a solid fatty material

(30) Priorité: 28.10.2005 FR 0553286
(43) Date de publication de la demande: 02.05.2007
(73) Titulaire: L'Oréal, 75008 Paris (FR)
(72) Inventeur: Mezure, Patricia, 78380 Bougival (FR); Lazzeri, Pascale, 92300 Levallois Perret (FR)
(74) Mandataire: Le Blainvaux Bellegarde, Françoise

(56) Documents cités:
- EP-A- 1 132 079
- FR-A- 2 847 831
- US-A1- 2004 223 938
- PATENT ABSTRACTS OF JAPAN vol. 1997, no. 10, 31 octobre 1997 (1997-10-31) & JP 09 165320 A (KAO CORP), 24 juin 1997 (1997-06-24)

## Description

La présente invention est relative à une composition cosmétique notamment de conditionnement des cheveux comprenant au moins un ester de sorbitan oxyéthyléné, au moins un polymère cationique non siliconé et au moins un corps gras solide non siliconé et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques comprenant tensioactifs cationiques et des polysaccharides épaississants tels que notamment l'amidon ou les celluloses.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

De plus les soins utilisés pour les cheveux très sensibilisés peuvent être insuffisant pour traiter les pointes qui sont le plus souvent très abîmées.

En résumé, il s'avère que les compositions cosmétiques actuelles de conditionnement, ne donnent pas complètement satisfaction. Ainsi, on cherche à obtenir des compositions cosmétiques ayant de très bonnes propriétés cosmétiques en particulier sur des cheveux très sensibilisés.

La demanderesse a maintenant découvert que l'association d'au moins un ester de sorbitan oxyéthyléné particulier, d'un polymère cationique non siliconé de charge cationique particulière et d'un composé gras solide non siliconé permet de remédier à ces inconvénients.

Les cheveux traités avec cette composition sont lisses, se démêlent facilement, sont brillants, souples, individualisés et ont un toucher doux et sans résidus. Les cheveux ont un aspect naturel et non chargé. Le lissage est homogène des racines aux pointes. Les pointes présentent moins de fourches.
De plus, ces effets perdurent dans le temps.

La titulaire a découvert que l'adjonction d'ester de sorbitan faiblement oxyéthyléné, susdéfini, permettait de réduire de façon surprenante les réactions d'inconfort (démangeaisons, rougeurs ...), notamment au niveau du cuir chevelu, des compositions contenant des tensioactifs susceptibles de provoquer ce type de réactions lorsqu'ils sont utilisés seuls.

Ainsi, selon la présente invention, il est maintenant proposé de nouvelles compositions cosmétiques, comprenant, dans un milieu aqueux cosmétiquement acceptable, au moins un ester de sorbitan oxyéthyléné et d'acide gras en C₈-C_{30,} saturé ou insaturé, linéaire ou ramifié et ayant un nombre de moles d'oxyde d'éthylène inférieur ou égal à 20, au moins un polymère cationique non siliconé présentant une densité de charge cationique allant de 4 meq/g à 8 meq/g et de nature chimique définie et au moins un corps gras solide non siliconé.

Un autre objet de l'invention consiste en un procédé de traitement cosmétique des matières kératiniques, en particulier les cheveux mettant en oeuvre la composition susvisée.

D'autres objets, caractéristiques, aspects et avantages de l'invention apparaîtront encore plus clairement à la lecture de la description et des divers exemples qui suivent.

Par cheveux sensibilisés, on comprend selon la présente invention, des cheveux ayant subi des agressions extérieures physiques (lumière, chaleur, ondes...), des agressions mécaniques (brushing, peignages ou brossages répétés...) ou des agressions chimiques (coloration d'oxydation, décoloration, permanente, défrisage...). Parmi ces agressions, on notera le caractère particulièrement délétère des agressions chimiques. Les compositions selon l'invention sont particulièrement efficaces sur les cheveux sensibilisés par des agressions chimiques.

Par « au moins un », on comprendra « un ou plusieurs », C'est-à-dire un, deux, trois ou plus.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Par corps gras non siliconé on entend tout composé organique comportant dans sa structure au moins une chaine hydocarboné comportant au moins 10 atomes de carbone, ne comportant pas d'atomes de silicium.

Les corps gras solides présentent de préférence un point de fusion supérieur ou égal à 35°C et/ou présente une viscosité à la température de 40°C et sous un taux de cisaillement de 1s⁻¹, supérieure ou égale à 1 Pa.s.

Les corps gras solides selon l'invention sont de préférence non polymériques, c'est-à-dire qu'ils ne comportent pas d'unités monomériques répétées autres que des unités oxyde d'alkylène.

Les acides gras des esters de sorbitan et d'acide gras en C₈ -C₃₀, ayant un nombre de moles d'oxyde d'éthylène inférieur ou égal à 20 ont de préférence de 8 à 24 atomes de carbone et plus particulièrement de 8 à 18 atomes de carbone. Les acides gras sont notamment choisis parmi l'acide laurique, l'acide palmitique, l'acide oléique et l'acide stéarique, et de préférence parmi l'acide laurique et l'acide stéarique et encore plus particulièrement l'acide laurique.

On utilise de préférence des mono-esters d'acide gras en C8-C24 et de sorbitan oxyéthyléné. Le nombre de moles d'oxyde d'éthylène est de préférence inférieur à 10 et va plus particulièrement de 3 à 8 moles d'oxyde d'éthylène et en particulier est égal à 4.
Les esters de sorbitan préférés sont le mono-laurate de sorbitan oxyéthyléné à 4 moles d'oxyde d'éthylène (4 OE) ou polysorbate 21, le mono-stéarate de sorbitan oxyéthyléné à 4 moles d'oxyde d'éthylène (4 OE) ou polysorbate 61, le mono-oléate de sorbitan oxyéthyléné à 5 moles d'oxyde d'éthylène (5 OE) ou polysorbate 81.
Le polysorbate 21 est particulièrement préféré et est notamment commercialisé sous la dénomination TWEEN 21 par la société UNIQEMA.

Selon l'invention, la composition peut avantageusement comprendre des mélanges d'esters de sorbitan oxyéthylénés et notamment du polysorbate 21 avec du polysorbate 20 (monolaurate de sorbitan oxyéthyléné à 20 OE).

Selon la présente invention, l'ester de sorbitan oxyéthyléné peut être présent dans la composition cosmétique dans des proportions allant de 0,1 à 10 %, et préférentiellement de 0,5 à 5 % en poids par rapport au poids total de ladite composition.

La composition cosmétique selon l'invention comprend un ou plusieurs polymères cationiques dont la densité de charge cationique est de 4 à 8 meq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl.

De manière générale, au sens de la présente invention, l'expression "polymère cationique" désigne tout polymère comprenant des groupes cationiques et/ou des groupes ionisables en groupes cationiques.

Les polymères cationiques utilisés dans le cadre de la présente invention, sont choisis parmi les homopolymères d'halogénure de dialkyldiallylammonium, les polycondensats à motifs récurrents diammonium quaternaire et les polymères réticulés de sels de méthacryloyloxyalkyl(C₁-C₄) trialkyl(C₁-C₄)ammonium.

Les polymères cationiques décrits ci-dessus sont présents de préférence en une quantité allant de 0,01 à 10 % en poids, mieux encore de 0,05 à 5 % en poids, et encore plus préférentiellement de 0,1 à 3 % en poids par rapport au poids total de la composition.

Les corps gras solides non siliconés selon l'invention peuvent être cristallisés, amorphes ou pateux.
Le point de fusion va de préférence de 35 à 250°C et plus particulièrement de 40 à 150°C.
Ces solides ont une viscosité à la température de 40°C et sous un taux de cisaillement de 1s⁻¹, allant de 1 Pa.s à 1000000 Pa.s et de préférence de 10 à 1000 Pa.s.

Les mesures de viscosités peuvent être réalisées à une température d'environ 40°C, sur un Carri-Med CSL2-500.

Les points de fusion peuvent être mesurés par DSC ou sur un banc Kofler. Le point de fusion peut être mesuré par analyse calorimétrique différentielle (DSC) avec une vitesse de montée en température de 10 °C/min. Le point de fusion est alors la température correspondant au sommet du pic endotherme de fusion obtenu lors de la mesure.

Les corps gras solides non siliconés présentant un point de fusion supérieur ou égal à 35 °c sont notamment choisis parmi les alcools gras oxyéthylénés ou non, les esters gras, les cires minérales et les cires organiques différentes des esters gras et des alcools gras et leurs mélanges.

Les alcools gras selon l'invention sont de préférence linéaires et saturés, et comportent de 12 à 40 atomes de carbone.

Les alcools gras présentent de préférence la structure R-OH, dans laquelle R désigne de préférence un groupement alkyle en C12-C24. R peut être substitué par un ou plusieurs groupements hydroxy et de préférence non substitué.

A titre d'exemple on peut citer l'alcool myristique l'alcool cétylique, l'alcool stéarylique, l'alcool béhénylique et leurs mélanges.
L'alcool gras peut représenter un mélange d'alcools gras, ce qui signifie que dans un produit commercial peuvent coexister plusieurs espèces d'alcools gras, sous forme d'un mélange.
De préférence, les alcools gras de l'invention sont non oxyalkylénés et/ou non glycérolés. Ces alcools gras peuvent être des constituants des cires animales ou végétales.

Les esters gras sont des esters comportant au moins 10 atomes de carbone et de préférence des esters d'un acide carboxylique comportant au moins 10 atomes de carbone et d'un monoalcool ou d'un polyol. Les esters gras selon l'invention peuvent être des mono, des di ou des triesters.
Les acides carboxyliques ont de préférence de 10 à 30 atomes de carbone et plus particulièrement de 12 à 24 atomes de carbone. Les alcools ont de préférence de 10 à 30 atomes de carbone et plus particulièrement de 12 à 24 atomes de carbone. De préférence, les esters gras solides selon l'invention sont des esters d'acide gras monocarboxylique comportant au moins 10 atomes de carbone et d'un monoalcool comportant au moins 10 atomes de carbone.

A titre d'esters selon l'invention, on peut citer le myristate de cétyle, le myristate de myristyle, le palmitate de palmityle, le palmitate de stéaryle, le stéarate de palmityle le stéarate de stéaryle et leurs mélanges.

Les esters gras peuvent être des constituants des cires animales ou végétales.
Une cire, au sens de la présente invention, est un composé lipophile, solide à température ambiante (environ 25°C), à changement d'état solide/liquide réversible, ayant une température de fusion supérieure à environ 40°C et pouvant aller jusqu'à 200°C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).
A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, les ozokérite, la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, et leurs mélanges. Comme cires organiques, on peut aussi citer les cires comprenant des fonctions amides et en particulier les céramides naturelles ou synthétiques.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina); d'autres cires ou matières premières cireuses utilisables selon l'invention sont notamment les cires marines telles que celle vendue par la Société SOPHIM sous la référence M82.

Le ou les corps gras solides non siliconés peuvent être présents dans la composition à une teneur allant de 0,1 à 10%, de préférence de 0,5 à 5%, encore de préférence de 1 à 4% en poids du poids total de la composition.

La composition selon l'invention peut contenir éventuellement des tensioactifs.

Les tensioactifs peuvent être présents en une quantité allant de 0,1% à 10% en poids environ, de préférence de 0,5% à 8% et encore plus préférentiellement de 1% à 5%, par rapport au poids total de la composition.
Les tensioactifs additionnels sont de préférence choisis parmi les tensioactifs non ioniques et cationiques.

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.
Selon l'invention, les tensioactifs cationiques sont de préférence non polymériques.
A titre d'amines grasses, on peut notamment citer les alkyl amido amines telles que par exemple les alkyl (C8-C30) amido dialkyl (C1-C6)amines et en particulier la stéaramido propyl diméthylamine (MACKINE 301 proposé par MAC INTYRE).
A titre de sels d'ammonium quaternaire, on peut notamment citer, par exemple :
- ceux qui présentent la formule générale (V) suivante : 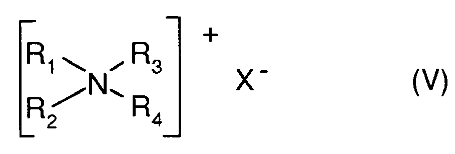 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C1-C30, , alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (Vl) suivante : 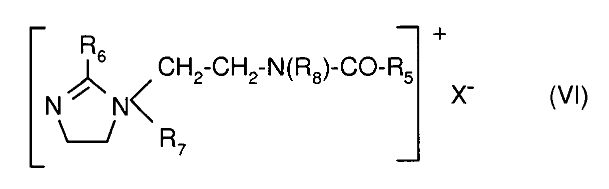 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 2002), le Quaternium-87 (CTFA 2002) ou le Quaternium-83 (CTFA 2002) commercialisés sous les dénominations "VARISOFT^{®}" W575PG par la société GOLDSCHMIDT,
- les sels de diammonium quaternaire de formule (VII) : 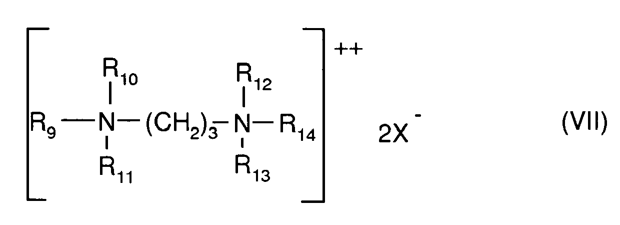 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire comprenant au moins une fonction ester, tels que ceux de formule (VIII) suivante : 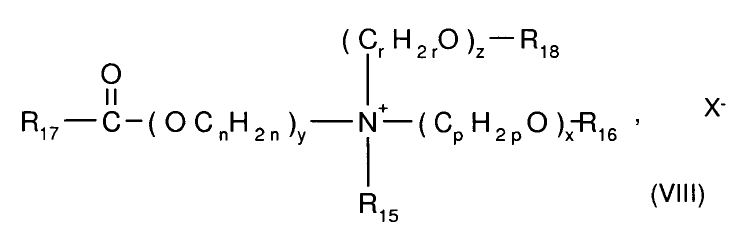 dans laquelle :
   R₁₅ est choisi parmi les radicaux alkyles en C₁-C₆ et les radicaux hydroxyalkyles ou dihydroxyalkyles en C₁-C₆;
   R₁₆ est choisi parmi :
      - le radical 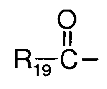
      - les radicaux R₂₀ hydrocarbonés en C₁-C₂₂, linéaires ou ramifiés, saturés ou insaturés,
      - l'atome d'hydrogène,
         R₁₇ est choisi parmi :
      - le radical 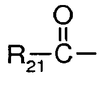
      - les radicaux R₂₂ hydrocarbonés en C₁-C₆, linéaires ou ramifiés, saturés ou insaturés,
      - l'atome d'hydrogène,
R₁₇, R₁₉ et R₂₁, identiques ou différents, sont choisis parmi les radicaux hydrocarbonés en C₇-C₂₁, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.
Les radicaux alkyles R₁₅ peuvent être linéaires ou ramifiés, et plus particulièrement linéaires.
De préférence, R₁₅ désigne un radical méthyle, éthyle, hydroxyéthyle ou dihydroxypropyle, et plus particulièrement un radical méthyle ou éthyle.
Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.
Lorsque R₁₈ est un radical R₂₂ hydrocarboné, il a de préférence 1 à 3 atomes de carbone.
Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
De préférence, x et z, identiques ou
différents, valent 0 ou 1.
Avantageusement, y est égal à 1.
De préférence, r, n et p, identiques ou différents, valent 2 ou 3, et encore plus particulièrement sont égaux à 2.

L'anion X⁻ est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X⁻ est encore plus particulièrement le chlorure ou le méthylsulfate.
On utilise plus particulièrement dans la composition selon l'invention, les sels d'ammonium de formule (IV) dans laquelle:
- R₁₅ désigne un radical méthyle ou éthyle,
- x et y sont égaux à 1;
- z est égal à 0 ou 1 ;
- r, n et pt sont égaux à 2 ;
- R₁₆ est choisi parmi :
- le radical 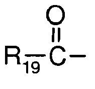
- les radicaux méthyle, éthyle ou hydrocarbonés en C₁₄-C₂₂,
- l'atome d'hydrogène ;
- R₁₈ est choisi parmi :
- le radical 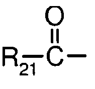
- l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.
Avantageusement, les radicaux hydrocarbonés sont linéaires.

On peut citer par exemple les composés de formule (Vlll) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.
Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.
De tels composés sont par exemple commercialisés sous les dénominations DEHYQUART^{®} par la société COGNIS, STEPANQUAT^{®} par la société STEPAN, NOXAMIUM^{®} par la société CECA, REWOQUAT^{®} WE 18 par la société REWO-GOLDSCHMIDT.
La composition selon l'invention peut contenir de préférence un mélange de sels de mono-, di- et triester d'ammonium quaternaire avec une majorité en poids de sels de diester.
Comme mélange de sels d'ammonium, on peut utiliser par exemple le mélange comprenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium comprenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium correspondant au QUATERNIUM-70 (CTFA 2002) commercialisé sous la dénomination CERAPHYL^{®} 70 par la société ISP.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium et le chlorure de palmitylamidopropyltriméthylammonium.

Lorsqu'ils sont présents, la composition selon l'invention comprend de préférence le ou les tensioactifs cationiques en une quantité allant de 0,05 à 10 % en poids, de préférence de 0,1 à 8 % en poids par rapport au poids total de la composition et plus particulièrement de 0,2 à 5% en poids.

Les compositions selon l'invention sont de préférence des compositions non lavantes (non détergentes), elles comprennent de préférence moins de 4% en poids de tensioactifs anioniques et plus particulièrement moins de 1% en poids par rapport au poids total de la composition.

La composition selon l'invention peut comprendre en outre au moins un agent conditionneur additionnel choisi parmi les silicones, les polymères cationiques différents des polymères cationiques selon l'invention, les esters gras carboxyliques différents de ceux de l'invention, les huiles végétales, les huiles minérales, les huiles de synthèse telles que les poly(alpha-oléfines), et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 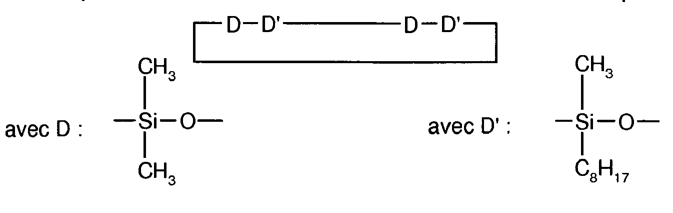 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701 ^{E} de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201 et "ABIL^{®} S255";
- des groupements hydroxyacylamino, comme les polyorgano-siloxanes décrits dans la demande EP 342 834. On peut citer, par exemple, le produit Q2-8413 de la société DOW CORNING.
A titre d'exemples de silicones, on utilise de préférence les polydiméthylsiloxanes, les polyalkylarylsiloxanes, les polydiméthylsiloxanes à groupements aminés ou alcoxylés.

La composition selon l'invention peut également comprendre un ou plusieurs esters liquides d'acide carboxylique, tels que par exemple, l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le lactate de 2-octyldodécyle, le néopentanoate d'isostéaryle, le néopentanoate de tridécyle, le néopentanoate d'isocétyle et le néopentanoate d'isoarachidyle et leurs mélanges.

La composition selon l'invention peut encore comprendre une ou plusieurs huiles végétales telles que l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

Comme huiles minérales additionnelles, on peut notamment citer l'huile de paraffine et l'huile de vaseline.

Les agents conditionneurs additionnels sont contenus de préférence dans la composition selon l'invention en une quantité allant de 0,01 % à 20 % en poids, mieux encore allant de 0,1 % à 10 % en poids et plus particulièrement allant de 0,3 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀ ; et leurs mélanges.. Les solvants sont de préférence choisis parmi la glycérine et le propylène glycol.

Le milieu cosmétiquement acceptable notamment aqueux représente de préférence de 30 à 98% en poids par rapport au poids total de la composition.

Les solvants sont de préférence présents dans des concentrations allant de 0,5 à 30% en poids par rapport au poids total de la composition.

Le pH des compositions de l'invention est compris de préférence de 2 à 8, de préférence de 3 à 7.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux sensibilisés. Cet après-shampooing peut être rincé ou non rincé et de préférence rincé.

Les compositions cosmétiques selon l'invention peuvent se présenter sous forme de gel, de lait, de crème, d'émulsion, de lotions fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.
Le rinçage s'effectue par exemple avec de l'eau.

Ainsi, ce procédé selon l'invention permet le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 A 8

On a préparé les compositions suivantes d'après-shampooing à rincer :
1. Dans la cuve de fabrication introduire la totalité de l'eau, ajouter les composés hydrosolubles à l'exception de l'ester de sorbitan oxyéthyléné. Chauffer jusqu'à 80°C sous agitation raclante jusqu'à complète dissolution.
2. Dans une cuve annexe introduire les composés lipophiles hormis les éventuelles silicones et les parfums. Chauffer jusqu'à 80°C
3. Introduire le contenu de l'annexe et les éventuelles silicones dans la cuve de fabrication et émulsionner 10 mn sous forte agitation turbine et raclante en maintenant la température. Puis commencer le refroidissement.
4. à 30°C introduire l'ester de sorbitan oxyéthyléné et le parfum sous agitation raclante.

| Exemple 1 | en g MA |
|---|---|
| Copolymère SMDI (Saturated Methylene diphenyl Diisocyanate)/ polyéthylène glycol à terminaison alkyle (méthyl/C18) à 15% MA (ACULYN 46 de ROHM & HAAS) | 0,45 |
| Homopolymère de chlorure de méthacrylate d'éthyl triméthyl ammonium reticulé à 50% MA (SALCARE SC 95 de CIBA) | 0,55 |
| Alcool cétylstéarylique (LANETTE O OR de COGNIS) | 2,5 |
| Monolaurate de sorbitan oxyéthyléné à 4 moles d'oxyde d'éthylène (4 OE) (TWEEN 21 de UNIQEMA) | 4 |
| Parfum | qs |
| Conservateurs | qs |
| Eau qsp | 100 g |

### Exemple 2 :

| | en g MA |
|---|---|
| Copolymère SMDI (Saturated Methylene diphenyl Diisocyanate)/ polyéthylène glycol à terminaison alkyle (méthyl/C18) à15% MA (ACULYN 46 de ROHM & HAAS) | 0,6 |
| Homopolymère de chlorure de méthacrylate d'éthyl triméthyl ammonium reticulé à 50% MA (SALCARE SC 95 de CIBA) | 0,55 |
| Alcool cétylstéarylique (LANETTE O OR de COGNIS) | 2 |
| Monolaurate de sorbitan oxyéthyléné à 4 moles d'oxyde d'éthylène (4 OE) (TWEEN 21 de UNIQEMA) | 4 |
| Piroctone olamine (Octopyrox de CLARIANT) | 0,3 |
| Parfum | qs |
| Conservateurs | qs |
| Eau qsp | 100 g |

### Exemple 3

| | en g MA |
|---|---|
| Copolymère SMDI (Saturated Methylene diphenyl Diisocyanate)/ polyéthylène glycol à terminaison alkyle (méthyl/C18) à15% MA (ACULYN 46 de ROHM & HAAS) | 0,45 |
| Homopolymère de chlorure de méthacrylate d'éthyl triméthyl ammonium reticulé à 50% MA (SALCARE SC 95 de CIBA) | 0,55 |
| Alcool cétylstéarylique (LANETTE O OR de COGNIS) | 2 |
| Monolaurate de sorbitan oxyéthyléné à 4 moles d'oxyde d'éthylène (4 OE) (TWEEN 21 de UNIQEMA) | 4 |
| Aminexil | 0,3 |
| Parfum | qs |
| Conservateurs | qs |
| Eau qsp | 100 g |

### Exemple 4

| | en g MA |
|---|---|
| Copolymère SMDI (Saturated Methylene diphenyl Diisocyanate)/ polyéthylène glycol à terminaison alkyle (méthyl/C18) à 15% MA (ACULYN 46 de ROHM & HAAS) | 0,45 |
| Homopolymère de chlorure de méthacrylate d'éthyl triméthyl ammonium reticulé à 50% MA (SALCARE SC 95 de CIBA) | 0,55 |
| Alcool cétylstéarylique (LANETTE O OR de COGNIS) | 2 |
| Monolaurate de sorbitan oxyéthyléné à 20 moles d'oxyde d'éthylène (20 OE) (TWEEN 20 de UNIQEMA) | 4 |
| Diaminopyrimidine oxide (Mexoryl sag de CHIMEX) | 1,5 |
| Parfum | qs |
| Conservateurs | qs |
| Eau qsp | 100 g |

### Exemple 5 : après shampooings rincé

| | en g MA |
|---|---|
| Copolymère SMDI (Saturated Methylene diphenyl Diisocyanate)/ polyéthylène glycol à terminaison alkyle (méthyl/C18) à 15% MA (ACULYN 46 de ROHM & HAAS) | 0,45 |
| Chlorure de poly di-méthyl di-allyl ammonium à 40% MA (MERQUAT 100 de NALCO) | 0,4 |
| Alcool cétylstéarylique (LANETTE O OR de COGNIS) | 2 |
| Monolaurate de sorbitan oxyéthyléné à 20 moles d'oxyde d'éthylène (20 OE) (TWEEN 20 de UNIQEMA) | 4 |
| Parfum | qs |
| Conservateurs | qs |
| Eau qsp | 100 g |

### Exemple 6 d'après-shampooings à rincer :

| | en g MA |
|---|---|
| Copolymère SMDI (Saturated Methylene diphenyl Diisocyanate)/ polyéthylène glycol à terminaison alkyle (méthyl/C18) à15% MA (ACULYN 46 de ROHM & HAAS) | 0,45 |
| Chlorure de poly di-méthyl di-allyl ammonium à 40% MA (MERQUAT 100 de NALCO) | 0,4 |
| Alcool cétylstéarylique (LANETTE O OR de COGNIS) | 2 |
| Monolaurate de sorbitan oxyéthyléné à 4 moles d'oxyde d'éthylène (4 OE) (TWEEN 21 de UNIQEMA) | 4 |
| Parfum | qs |
| Conservateurs | qs |
| Eau qsp | 100 g |

### Exemple 7 d'après shampooing rincé :

| | en g ma |
|---|---|
| Cire de candelilla | 0,4 |
| Alcool stéarylique (LANETTE 18 de COGNIS) | 2 |
| Chlorure de behenyl triméthyl ammonium à 80% de MA (GENAMINE KDMP de CLARIANT) | 0,5 |
| Polycondensat de tétraméthyl hexaméthylènediamine en solution aqueuse à 60% MA (MEXOMER PO de CHIMEX) | 0,4 |
| Phosphate de di-amidon de maïs hydroxypropylé et prégélatinisé (STRUCTURE ZEA de NATIONAL STARCH) | 1,5 |
| Monolaurate de sorbitan oxyéthyléné à 4 moles d'oxyde d'éthylène (4 OE) (TWEEN 21 de UNIQEMA) | 4 |
| Parfum | qs |
| Conservateurs | qs |
| Eau qsp | 100 g |

### Exemple 8

| | EN g MA |
|---|---|
| Copolymère SMDI (Saturated Methylene diphenyl Diisocyanate)/ polyéthylène glycol à terminaison alkyle (méthyl/C18) à15% MA (ACULYN 46 de ROHM & HAAS) | 0,45 |
| Homopolymère de chlorure de méthacrylate d'éthyl triméthyl ammonium reticulé à 50% MA (SALCARE SC 95 de CIBA) | 0,55 |
| Alcool cétylstéarylique (LANETTE O OR de COGNIS) | 2,5 |
| Mono-stéarate de sorbitan oxyéthyléné à 20 OE (TWEEN 60 DE UNIQEMA) | 2 |
| Parfum | qs |
| Conservateurs | qs |
| Eau qsp | 100 g |

Ces compositions ont été appliquées sur des cheveux très sensibilisés. Les propriétés cosmétiques (démêlage, lissage, souplesse) sont excellentes et homogènes des racines aux pointes des cheveux. Les pointes ne sont pas fourchues.

Entre deux applications, les cheveux restent doux, souples et lisses.

## Revendications

1. Composition cosmétique, comprenant, dans un milieu aqueux cosmétiquement acceptable, au moins un ester de sorbitan oxyéthyléné et d'acide gras en C₈-C₃₀ saturé ou insaturé, linéaire ou ramifié et ayant un nombre de moles d'oxyde d'éthylène inférieur ou égal à 20, au moins un polymère cationique non siliconé présentant une densité de charge cationique allant de 4 à 8 meq/g et choisi parmi les homopolymères d'halogénure de dialkyldiallylammonium, les polycondensats à motifs récurrents diammonium quaternaire, et les polymères réticulés de sels de méthacryloyloxyalkyl(C₁-C₄) trialkyl(C₁-C₄)ammonium et au moins un corps gras solide non siliconé.

2. Composition selon la revendication 1, **caractérisée en ce que** les acides gras des esters de sorbitan et d'acide gras en C₈-C₃₀, ayant un nombre de moles d'oxyde d'éthylène inférieur ou égal à 20 ont de 8 à 24 atomes de carbone et plus particulièrement de 8 à 18 atomes de carbone.

3. Composition selon la revendication précédente, **caractérisée en ce que** les acides gras des esters de sorbitan et d'acide gras en C₈-C₃₀, sont l'acide laurique et l'acide stéarique, de préférence l'acide laurique.

4. Composition selon l'une quelconque des revendications précédentes, **caractérisée en ce que** les esters de sorbitan oxyéthyléné sont des mono-esters d'acide gras en C8-C24 et de sorbitan oxyéthyléné.

5. Composition selon l'une quelconque des revendications précédentes **caractérisée en ce que** le nombre de moles d'oxyde d'éthylène est inférieur à 10 et va plus particulièrement de 3 à 8 moles d'oxyde d'éthylène et en particulier est égal à 4.

6. Composition selon l'une quelconque des revendications précédentes, **caractérisée en ce que** les esters de sorbitan oxyéthyléné sont choisis parmi le mono-laurate de sorbitan oxyéthyléné avec 4 moles d'oxyde d'éthylène (40E) et le mono-stéarate de sorbitan oxyéthyléné avec 4 moles d'oxyde d'éthylène (4OE).

7. Composition selon l'une quelconque des revendications précédentes, **caractérisée en ce que** l'ester de sorbitan oxyéthyléné est le mono-laurate de sorbitan oxyéthyléné avec 4 moles d'oxyde d'éthylène (4OE).

8. Composition selon l'une quelconque des revendications précédentes, **caractérisée en ce que** l'ester de sorbitan oxyéthyléné est présent dans la composition dans des proportions allant de 0,1 à 10 %, et préférentiellement de 0,5 à 5 % en poids par rapport au poids total de ladite composition.

9. Composition selon l'une quelconque des revendications précédentes, **caractérisée en ce que** le polymère cationique est présent à une concentration allant de 0,01 % à 10 % en poids par rapport au poids total de la composition, de préférence de 0,05 % à 5 % en poids.

10. Composition selon l'une quelconque des revendications précédentes, **caractérisée en ce que** le corps gras solide est choisi parmi les alcools gras oxyéthyléné ou non, les esters gras, les cires minérales, les cires organiques différentes des esters gras et des alcools gras, et leurs mélanges.

11. Composition selon l'une quelconque des revendications précédentes, **caractérisée en ce que** le corps gras solide est une cire végétale ou animales.

12. Composition selon l'une quelconque des revendications précédentes, **caractérisée en ce que** le corps gras solide est un alcool gras linéaire.

13. Composition selon l'une quelconque des revendications précédentes, **caractérisée en ce que** le corps gras solide est un alcool gras saturé.

14. Composition selon l'une quelconque des revendications précédentes, **caractérisé en ce que** le corps gras solide est un alcool gras présentant la structure R-OH, dans laquelle R désigne un radical alkyle comportant de 12 à 24 atomes de carbone éventuellement hydroxylé.

15. Composition selon l'une quelconque des revendications précédentes, **caractérisé en ce que** le corps gras solide est un alcool gras choisi parmi l'alcool myristique, l'alcool cétylique, l'alcool béhénylique, l'alcool stéarylique, et leurs mélanges.

16. Composition selon la revendication 10, **caractérisée en que** les esters gras sont des esters d'un acide carboxylique et d'un monoalcool ou d'un polyol, l'ester comportant au moins 10 atomes de carbone.

17. Composition selon la revendication 16, **caractérisée en ce que** les esters gras sont choisis parmi des esters d'acide gras monocarboxylique comportant au moins 10 atomes de carbone et d'un monoalcool comportant au moins 10 atomes de carbone.

18. Composition selon la revendication 17, **caractérisée en ce que** les esters gras sont choisis parmi le myristate de cétyle, le myristate de myristyle, le palmitate de palmityle, le palmitate de stéaryle, le stéarate de palmityle le stéarate de stéaryle et leurs mélanges.

19. Composition selon l'une quelconque des revendications 10, **caractérisée en ce que** les cires sont choisies parmi la cire d'abeille, le spermaceti, la cire de lanoline et les dérivés de lanoline ; la cire de Carnauba, de Candellila, d'Ouricury, du Japon, le beurre de cacao ou les cires de fibres de liège ou de canne à sucre ; les cires de paraffine, de vaseline, de lignite ou les cires microcristallines, les ozokérite, la cire d'olivier, la cire de riz, la cire de jojoba hydrogénée ou les cires absolues de fleurs, les cires d'abeilles, ou les cires d'abeilles modifiées (cerabellina), les céramides naturelles ou synthétiques et leurs mélanges.

20. Composition selon l'une quelconque des revendications précédentes, **caractérisée par le fait que** le corps gras solide est présent dans les composition de l'invention à une concentration comprise entre 0,1 et 10% en poids, de préférence entre 0,5 et 5% par rapport au poids total de la composition.

21. Composition selon l'une quelconque des revendications précédentes, **caractérisée en qu'**elle comprend en outre un tensioactif cationique.

22. Composition selon la revendication précédente, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

23. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le Quaternium-87, le chlorure de béhénylamidopropyl 2,3-dihydroxypropyl diméthyl ammonium et le chlorure de palmitylamidopropyltriméthylammonium.

24. Composition selon l'une quelconque des revendications précédentes, **caractérisée en ce que** les tensioactifs cationiques sont contenus en une quantité allant de 0,05 à 10 % en poids, de préférence de 0,1 à 8 % en poids par rapport au poids total de la composition.

25. Composition selon l'une quelconque des revendications précédentes, **caractérisée en ce qu'**elle comprend en outre au moins un agent conditionneur additionnel.

26. Composition selon la revendication 25, **caractérisée en ce que** l'agent conditionneur additionnel est choisi parmi les silicones, les polymères cationiques différents des polymères cationiques selon l'invention, les esters carboxyliques liquides, les huiles végétales, les huiles minérales, les huiles de synthèse et leurs mélanges.

27. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

28. Composition selon la revendication 27, **caractérisée en ce que** le solvant cosmétiquement acceptable est choisi parmi les alcools inférieur en C₁-C₄ tel que l'éthanol, l'isopropanol, le tertio-butanol, le n-butanol ; les alkylèneglycols comme le propylèneglycol, les éthers de polyol ; les alcanes en C₅-C₁₀ et leurs mélanges.

29. Composition selon l'une quelconque des revendications précédentes, **caractérisée en ce qu'**elle comprend en outre des additifs tels que des polymères anioniques, non ioniques ou amphotères, des épaississants comme des acides ou des électrolytes, des opacifiants, des agents nacrants, des vitamines, des provitamines telles que le panthénol, des parfums, des colorants, des particules organiques ou minérales, des conservateurs, des agents de stabilisation du pH.

30. Composition selon l'une quelconque des revendications précédentes, **caractérisée par le fait qu'**elle se présente sous forme d'après-shampooing, de composition pour la permanente, le défrisage, la coloration ou la décoloration des cheveux, de composition à rincer à appliquer entre les deux étapes d'une permanente ou d'un défrisage.

31. Composition selon l'une quelconque des revendications précédentes, **caractérisée en ce qu'**elle est se présente sous la forme d'un après-shampoing à rincer.

32. Procédé de traitement cosmétique des matières kératiniques, telles que les cheveux, **caractérisé en ce qu'**il consiste à appliquer sur lesdites matières une quantité efficace d'une composition cosmétique selon l'une des revendications 1 à 31, puis à effectuer éventuellement un rinçage. 3

## Claims

1. Cosmetic composition comprising, in a cosmetically acceptable aqueous medium, at least one ester of oxyethylenated sorbitan and of a saturated or unsaturated, linear or branched C₈-C₃₀ fatty acid, with a number of moles of ethylene oxide of less than or equal to 20, at least one non-silicone cationic polymer with a cationic charge density ranging from 4 to 8 meq/g and chosen from dialkyldiallylammonium halide homopolymers, polycondensates containing diquaternary ammonium repeating units, and crosslinked polymers of methacryloyloxy(C₁-C₄)alkyltri(C₁-C₄)alkylammonium salts, and at least one non-silicone solid fatty substance.

2. Composition according to Claim 1, **characterized in that** the fatty acids of the esters of sorbitan and of a C₈-C₃₀ fatty acid, with a number of moles of ethylene oxide of less than or equal to 20, contain from 8 to 24 carbon atoms and more particularly from 8 to 18 carbon atoms.

3. Composition according to the preceding claim, **characterized in that** the fatty acids of the esters of sorbitan and of a C₈-C₃₀ fatty acid are lauric acid and stearic acid, preferably lauric acid.

4. Composition according to any one of the preceding claims, **characterized in that** the oxyethylenated sorbitan esters are monoesters of a C₈-C₂₄ fatty acid and of oxyethylenated sorbitan.

5. Composition according to any one of the preceding claims, **characterized in that** the number of moles of ethylene oxide is less than 10 and more particularly ranges from 3 to 8 mol of ethylene oxide, and in particular is equal to 4 mol.

6. Composition according to any one of the preceding claims, **characterized in that** the oxyethylenated sorbitan esters are chosen from sorbitan monolaurate oxyethylenated with 4 mol of ethylene oxide (4 EO) and sorbitan monostearate oxyethylenated with 4 mol of ethylene oxide (4 EO).

7. Composition according to any one of the preceding claims, **characterized in that** the oxyethylenated sorbitan ester is sorbitan monolaurate oxyethylenated with 4 mol of ethylene oxide (4 EO).

8. Composition according to any one of the preceding claims, **characterized in that** the oxyethylenated sorbitan ester is present in the composition in proportions ranging from 0.1% to 10% and preferentially from 0.5% to 5% by weight relative to the total weight of the said composition.

9. Composition according to any one of the preceding claims, **characterized in that** the cationic polymer is present at a concentration ranging from 0.01% to 10% and preferably from 0.05% to 5% by weight relative to the total weight of the composition.

10. Composition according to any one of the preceding claims, **characterized in that** the solid fatty substance is chosen from oxyethylenated or non-oxyethylenated fatty alcohols, fatty esters, mineral waxes, and organic waxes other than the fatty esters and fatty alcohols, and mixtures thereof.

11. Composition according to any one of the preceding claims, **characterized in that** the solid fatty substance is a plant or animal wax.

12. Composition according to any one of the preceding claims, **characterized in that** the solid fatty substance is a linear fatty alcohol.

13. Composition according to any one of the preceding claims, **characterized in that** the solid fatty substance is a saturated fatty alcohol.

14. Composition according to any one of the preceding claims, **characterized in that** the solid fatty substance is a fatty alcohol having the structure R-OH, in which R denotes an optionally hydroxylated alkyl radical containing from 12 to 24 carbon atoms.

15. Composition according to any one of the preceding claims, **characterized in that** the solid fatty substance is a fatty alcohol chosen from myristyl alcohol, cetyl alcohol, behenyl alcohol and stearyl alcohol, and mixtures thereof.

16. Composition according to Claim 10, **characterized in that** the fatty esters are esters of a carboxylic acid and of a monoalcohol or a polyol, the ester containing at least 10 carbon atoms.

17. Composition according to Claim 16, **characterized in that** the fatty esters are chosen from esters of a monocarboxylic fatty acid containing at least 10 carbon atoms and of a monoalcohol containing at least 10 carbon atoms.

18. Composition according to Claim 17, **characterized in that** the fatty esters are chosen from cetyl myristate, myristyl myristate, palmityl palmitate, stearyl palmitate, palmityl stearate and stearyl stearate, and mixtures thereof.

19. Composition according to either of Claims 10 and 11, **characterized in that** the waxes are chosen from beeswax, spermaceti, lanolin wax and lanolin derivatives; carnauba wax, candelilla wax, ouricury wax, Japan wax, cocoa butter, cork fibre wax or sugarcane wax; paraffin wax, petroleum jelly wax, lignite wax or microcrystalline waxes, ozokerite, olive wax, rice wax, hydrogenated jojoba wax, the absolute waxes of flowers, beeswaxes or modified beeswaxes (cerabellina), and natural or synthetic ceramides, and mixtures thereof.

20. Composition according to any one of the preceding claims, **characterized in that** the solid fatty substance is present in the composition of the invention at a concentration of between 0.1% and 10% by weight and preferably between 0.5% and 5% by weight relative to the total weight of the composition.

21. Composition according to any one of the preceding claims, **characterized in that** it also comprises a cationic surfactant.

22. Composition according to the preceding claim, **characterized in that** the cationic surfactants are chosen from the salts of optionally polyoxyalkylenated primary, secondary or tertiary fatty amines and quaternary ammonium salts, and mixtures thereof.

23. Composition according to any one of the preceding claims, **characterized in that** the cationic surfactants are chosen from behenyltrimethylammonium chloride, cetyltrimethylammonium chloride, Quaternium-83, Quaternium-87, behenylamidopropyl 2,3-dihydroxypropyl dimethyl ammonium chloride and palmitylamidopropyltrimethylammonium chloride.

24. Composition according to any one of the preceding claims, **characterized in that** the cationic surfactants are contained in an amount ranging from 0.05% to 10% by weight and preferably from 0.1% to 8% by weight relative to the total weight of the composition.

25. Composition according to any one of the preceding claims, **characterized in that** it also comprises at least one additional conditioning agent.

26. Composition according to Claim 25, **characterized in that** the additional conditioning agent is chosen from silicones, cationic polymers other than the cationic polymers according to the invention, liquid carboxylic esters, plant oils, mineral oils and synthetic oils, and mixtures thereof.

27. Composition according to any one of the preceding claims, **characterized in that** the cosmetically acceptable aqueous medium consists of water or of a mixture of water and of a cosmetically acceptable solvent.

28. Composition according to Claim 27, **characterized in that** the cosmetically acceptable solvent is chosen from C₁-C₄ lower alcohols such as ethanol, isopropanol, tert-butanol or n-butanol; alkylene glycols, for instance propylene glycol; polyol ethers; C₅-C₁₀ alkanes, and mixtures thereof.

29. Composition according to any one of the preceding claims, **characterized in that** it also comprises additives such as anionic, nonionic or amphoteric polymers, thickeners such as acids or electrolytes, opacifiers, nacreous agents, vitamins, provitamins such as panthenol, fragrances, dyes, organic or mineral particles, preserving agents and pH stabilizers.

30. Composition according to any one of the preceding claims, **characterized in that** it is in the form of a hair conditioner, a composition for permanent-waving, relaxing, dyeing or bleaching the hair, or a rinse-out composition to be applied between the two steps of a permanent-waving or hair-relaxing operation.

31. Composition according to any one of the preceding claims, **characterized in that** it is in the form of a rinse-out hair conditioner.

32. Cosmetic process for treating keratin materials, such as the hair, **characterized in that** it consists in applying to the said materials an effective amount of a cosmetic composition according to one of Claims 1 to 31, optionally followed by rinsing.

## Patentansprüche

1. Kosmetische Zusammensetzung, die in einem kosmetisch akzeptablen wässrigen Medium mindestens einen Ester von ethoxyliertem Sorbitan und einer geradkettigen oder verzweigten, gesättigten oder ungesättigten C₅-₃₀-Fettsäure mit einer Molzahl von Ethylenoxid von kleiner oder gleich 20, mindestens ein nicht siliconiertes kationisches Polymer, das eine kationische Ladungsdichte von 4 bis 8 meq/g aufweist und unter den Homopolymeren von Dialkyldiallylammoniumhalogeniden, Polykondensaten mit wiederkehrenden quartären Diammoniumeinheiten und vernetzten Polymeren vom Methacryloyloxyalkyl(C₁₋₄)trialkyl(C₁₋₄)-ammoniumsalzen ausgewählt ist, und mindestens eine feste, nicht siliconierte Fettsubstanz enthält.

2. Zusammensetzung nach Anspruch 1, **dadurch gekennzeichnet, dass** die Fettsäuren der Ester von Sorbitan und einer C₈₋₃₀-Fettsäure mit einer Molzahl von Ethylenoxid von 20 oder darunter 8 bis 24 Kohlenstoffatome und insbesondere 8 bis 18 Kohlenstoffatome aufweisen.

3. Zusammensetzung nach dem vorhergehenden Anspruch, **dadurch gekennzeichnet, dass** die Fettsäuren der Ester von Sorbitan und einer C₈₋₃₀-Fettsäure unter Laurinsäure und Stearinsäure und vorzugsweise Laurinsäure ausgewählt sind.

4. Zusammensetzung nach einem der vorhergehenden Ansprüche, **dadurch gekennzeichnet, dass** die ethoxylierten Sorbitanester Monoester einer C₈₋₂₄-Fettsäure und ethoxyliertem Sorbitan sind.

5. Zusammensetzung nach einem der vorhergehenden Ansprüche, **dadurch gekennzeichnet, dass** die Ethylenoxid-Molzahl unter 10 und insbesondere im Bereich von 3 bis 8 mol Ethylenoxid liegt und insbesondere 4 beträgt.

6. Zusammensetzung nach einem der vorhergehenden Ansprüche, **dadurch gekennzeichnet, dass** die ethoxylierten Sorbitanester unter mit 4 mol Ethylenoxid (4 EO) ethoxyliertem Sorbitanmonolaizrat und mit 4 mol Ethylenoxid (4 EO) ethoxyliertem Sorbitanmonostearat ausgewählt sind.

7. Zusammensetzung nach einem der vorhergehenden Ansprüche, **dadurch gekennzeichnet, dass** es sich bei dem ethoxylierten Sorbitanester um das mit 4 mol Ethylenoxid (4 EO) ethoxylierte Sorbitanmonolaurat handelt.

8. Zusammensetzung nach einem der vorhergehenden Ansprüche, **dadurch gekennzeichnet, dass** der ethoxylierte Sorbitanester in der Zusammensetzung in Mengenanteilen von 0,1 bis 10 % und vorzugsweise 0,5 bis 5 Gew.-%, bezogen auf das Gesamtgewicht der Zusammensetzung, enthalten ist.

9. Zusammensetzung nach einem der vorhergehenden Ansprüche, **dadurch gekennzeichnet, dass** das kationische Polymer in Konzentrationen von 0,01 bis 10 Gew.-%, bezogen auf das Gesamtgewicht der Zusammensetzung, und vorzugsweise 0,5 bis 5 Gew.-% enthalten ist.

10. Zusammensetzung nach einem der vorhergehenden Ansprüche, **dadurch gekennzeichnet, dass** die feste Fettsubstanz unter den ethoxylierten oder nicht ethoxylierten Fettalkoholen, Fettsäureestern, Mineralwachsen, organischen Wachsen, die von Fettsäureestern und Fettalkoholen verschieden sind, und deren Gemischen ausgewählt ist.

11. Zusammensetzung nach einem der vorhergehenden Ansprüche, **dadurch gekennzeichnet, dass** die feste Fettsubstanz ein pflanzliches oder tierisches Wachs ist.

12. Zusammensetzung nach einem der vorhergehenden Ansprüche, **dadurch gekennzeichnet, dass** die feste Fettsubstanz ein linearer Fettalkohol ist.

13. Zusammensetzung nach einem der vorhergehenden Ansprüche, **dadurch gekennzeichnet, dass** die feste Fettsubstanz ein gesättigter Fettalkohol ist.

14. Zusammensetzung nach einem der vorhergehenden Ansprüche, **dadurch gekennzeichnet, dass** die feste Fettsubstanz ein Fettalkohol ist, der die Struktur R-OH besitzt, wobei R eine Alkylgruppe mit 12 bis 24 Kohlenstoffatomen bedeutet, die gegebenenfalls hydroxyliert ist.

15. Zusammensetzung nach einem der vorhergehenden Ansprüche, **dadurch gekennzeichnet, dass** die feste Fettsubstanz ein Fettalkohol ist, der unter Myristylalkohol, Cetylalkohol, Behenylalkohol, Stearylalkohol und deren Gemischen ausgewählt ist.

16. Zusammensetzung nach Anspruch 10, **dadurch gekennzeichnet, dass** die Fettsäureester Ester einer Carbonsäure und eines Monoalkohols oder eines Polyols sind, wobei der Ester mindestens 10 Kohlenstoffatome aufweist.

17. Zusammensetzung nach Anspruch 16, **dadurch gekennzeichnet, dass** die Fettsäureester unter den Estern einer Monocarbonsäure, die mindestens 10 Kohlenstoffatome aufweist, und eines Monoalkohols ausgewählt sind, der mindestens 10 Kohlenstoffatome aufweist.

18. Zusammensetzung nach Anspruch 17, **dadurch gekennzeichnet, dass** die Fettsäureester unter Cetylmyristat, Myristylmyristat, Palmitylpalmitat, Stearylpalmitat, Palmitylstearat, Stearylstearat und deren Gemischen ausgewählt ist.

19. Zusammensetzung nach Anspruch 10, **dadurch gekennzeichnet, dass** die Wachse unter Bienenwachs, Spermaceti, Lanolinwachs und den Lanolinderivaten; Carnaubawachs, Candelillawachs, Ouricurywachs, Japanwachs, Kakaobutter, Korkfaserwachs oder Zuckerrohrwachs; Paraffinwachs, Vaseline, Lignit oder mikrokristallinen Wachsen, Ozokerit, Ölbaumwachs, Reiswachs, hydriertem Jojobawachs oder reinen Blütenwachsen, Bienenwachsen oder modifizierten Bienenwachsen (Cera bellina), natürlichen oder synthetischen Ceramiden und deren Gemischen ausgewählt sind.

20. Zusammensetzung nach einem der vorhergehenden Ansprüche, **dadurch gekennzeichnet, dass** die feste Fettsubstanz in der Zusammensetzung in einer Konzentration von 0,1 bis 10 Gew.-% und vorzugsweise 0,5 bis 5 %, bezogen auf das Gesamtgewicht der Zusammensetzung, enthalten ist.

21. Zusammensetzung nach einem der vorhergehenden Ansprüche, **dadurch gekennzeichnet, dass** sie ferner einen kationischen grenzflächenaktiven Stoff enthält.

22. Zusammensetzung nach dem vorhergehenden Anspruch, **dadurch gekennzeichnet, dass** die kationischen grenzflächenaktiven Stoffe unter den Salzen von primären, sekundären oder tertiären Aminen, die gegebenenfalls polyalkoxyliert sind, quartären Ammoniumsalzen und deren Gemischen ausgewählt sind.

23. Zusammensetzung nach einem der vorhergehenden Ansprüche, **dadurch gekennzeichnet, dass** die kationischen grenzflächenaktiven Stoffe unter Behenyltrimethylammoniumchlorid, Cetyltrimethylammoniumchlorid, Quaternium-83, Quaternium-87, Behenylamidopropyl-2,3-dihydroxypropyldimethylammoniumchlorid und Palmitylamidopropyltrimethylammoniumchlorid ausgewählt sind.

24. Zusammensetzung nach einem der vorhergehenden Ansprüche, **dadurch gekennzeichnet, dass** die kationischen grenzflächenaktiven Stoffe in einem Mengenanteil im Bereich von 0,05 bis 10 Gew.-% und vorzugsweise 0,1 bis 8 Gew.-%, bezogen auf das Gesamtgewicht der Zusammensetzung, enthalten sind.

25. Zusammensetzung nach einem der vorhergehenden Ansprüche, **dadurch gekennzeichnet, dass** sie ferner ein ergänzendes Konditioniermittel enthält.

26. Zusammensetzung nach Anspruch 25, **dadurch gekennzeichnet, dass** das ergänzende Konditioniermittel unter den Siliconen, kationischen Polymeren, die von den erfindungsgemäßen kationischen Polymeren verschieden sind, flüssigen Fettsäureestern, pflanzlichen Ölen, Mineralölen, synthetischen Ölen und deren Gemischen ausgewählt ist.

27. Zusammensetzung nach einem der vorhergehenden Ansprüche, **dadurch gekennzeichnet, dass** das kosmetisch akzeptable wässrige Medium aus Wasser oder aus einem Gemisch von Wasser und einem kosmetisch akzeptablen Lösungsmittel besteht.

28. Zusammensetzung nach Anspruch 27, **dadurch gekennzeichnet, dass** das kosmetisch akzeptable Lösungsmittel unter den niederen Alkoholen mit 1 bis 4 Kohlenstoffatomen, wie Ethanol, Isopropanol, tert-Butanol, n-Butanol; Alkylenglycolen, wie Propylenglycol, Polyolethern; Alkanen mit 5 bis 10 Kohlenstoffatomen und deren Gemischen ausgewählt ist.

29. Zusammensetzung nach einem der vorhergehenden Ansprüche, **dadurch gekennzeichnet, dass** sie ferner Additive enthält, wie beispielsweise anionische, nichtionische oder amphotere Polymere, Verdickungsmittel, wie Säuren oder Elektrolyte, Trübungsmittel, Perlglanzstoffe, Vitamine, Provitamine wie Panthenol, Parfums, Farbmittel, organische oder anorganische Partikel, Konservierungsmittel oder pH-Stabilisatoren.

30. Zusammensetzung nach einem der vorhergehenden Ansprüche, **dadurch gekennzeichnet, dass** sie in Form einer Pflegespülung, als Zusammensetzung für dauerhafte Verformungen, zum Entkräuseln, zum Färben oder zum Entfärben der Haare, als Zusammensetzung, die ausgespült wird und zwischen den beiden Schritten einer dauerhaften Verformung oder einer Entkräuselung aufgebracht wird, vorliegt.

31. Zusammensetzung nach einem der vorhergehenden Ansprüche, **dadurch gekennzeichnet, dass** sie in Form eines Produktes, das nach der Haarwäsche aufgebracht und ausgespült wird, vorliegt.

32. Verfahren zur kosmetischen Behandlung von Keratinsubstanzen, wie Haaren, **dadurch gekennzeichnet, dass** es darin besteht, eine wirksame Menge einer kosmetischen Zusammensetzung nach einem der Ansprüche 1 bis 31 auf die Keratinsubstanzen aufzutragen und anschließend gegebenenfalls mit Wasser zu spülen.
